# EUROPÄISCHE PATENTSCHRIFT

(11) **EP 1 407 246 B1**
(45) Veröffentlichungstag und Bekanntmachung des Hinweises auf die Patenterteilung: **27.09.2006**
(21) Anmeldenummer: 02769131.0
(22) Anmeldetag: 02.05.2002
(51) Int. Cl.: G01N 1/28, G01N 33/18, B08B 11/00, B08B 17/00

(54) **VORRICHTUNG ZUR ENTNAHME VON ABWASSERPROBEN MIT EINER FILTERVORRICHTUNG, DIE ELEKTROLYTISCH GEREINIGT WIRD**
DEVICE FOR REMOVING WASTE WATER SAMPLES WITH A FILTER DEVICE WHICH IS ELECTROLYTICALLY CLEANED
DISPOSITIF DE PRELEVEMENT D'ECHANTILLONS D'EAUX USEES AVEC UN DISPOSITIF DE FILTRATION QUI EST NETTOYE ELECTROLYTIQUEMENT

(30) Priorität: 10.05.2001 DE 10122801
(43) Veröffentlichungstag der Anmeldung: 14.04.2004
(73) Patentinhaber: ISCO Inc., Lincoln Nebraska 68501 (US)
(72) Erfinder: BETTMANN, Oliver, 65439 Flörsheim (DE); TOTTEWITZ, Michael, 64807 Diebug (DE)
(74) Vertreter: Andres, Angelika Maria
(86) Internationale Anmeldenummer: PCT/EP2002/004811
(87) Internationale Veröffentlichungsnummer: WO 2002/090937

(56) Entgegenhaltungen:
- EP-A- 0 380 266
- EP-A- 0 474 365
- EP-A- 0 686 420
- EP-A- 1 094 311
- US-A- 4 624 760
- US-A- 5 167 802
- US-A- 5 958 242

## Beschreibung

Die Erfindung betrifft ein Verfahren zur Entnahme von Abwasserproben, insbesondere zur fotometrischen Analyse, wobei eine Filtratmenge durch eine Filtermatte hindurch angesaugt und daraus eine Probenmenge entnommen und einer Analyse zugeführt wird, wobei die Filtermatte außerhalb der Probenentnahmen intermittierend gereinigt wird.

Bei der Analyse von Abwasserproben müssen für die Probenentnahme die Feststoffbestandteile des Abwassers durch Filtration zurückgehalten werden. Bei allen angewandten Analyseverfahren können die Feststoffbestandteile die Analyseeinrichtung verstopfen. Bei der fotometrischen Analyse, bei der nach Zugabe eines Farbreagenz ein Farbumschlag optisch erfasst wird, würde eine durch Feststoffbestandteile verursachte Trübung der Abwasserprobe das Messergebnis verfälschen. Deshalb ist für die fotometrische Analyse von Abwasserproben eine besonders wirksame Filtrierung erforderlich.

Der Forderung nach hoher Filterfeinheit steht aber der Nachteil gegenüber, dass sich die erforderlichen feinen Filtermatten verhältnismäßig schnell zusetzen. Bei der Gewinnung von Abwasserproben besteht die besondere Schwierigkeit darin, dass eine einfache und übliche Filterrückspülung zur Reinigung der Filterfläche nicht ausreicht, weil die im Abwasser enthaltenen biologischen Keime zur Bildung eines biologischen Bewuchses auf der Filterfläche führen.

Ein solcher biologischer Bewuchs kann auch durch eine mechanische Filterreinigung nur unvollständig entfernt werden, insbesondere weil hierbei die feinen Filterporen nicht vollständig erreicht werden. Auch eine Reinigung unter Zugabe von chemisch wirkenden Reinigungeflüssigkeiten wirkt nur unzureichend und ist zeitaufwendig. Dies gilt in besonderem Maße bei Filtern mit hoher Filterfeinheit, wie sie bei der Gewinnung von Abwasserproben für die fotometrische Analyse erforderlich ist.

Das US Patent Nr. 4,624,760 offenbart ein Verfahren zum Reinigen eines porösen, elektrisch leitfähigen Filters während des Einsatzes des Filters zum Filtern eines wässrigen Prozessmediums durch Einrichten einer elektrochemischen Zelle mit dem Filter als erster Elektrode und einer Gegenelektrode sowie dem Prozessmedium als Elektrolyt, und Betreiben der Zelle unter Elektrolyse des Elektrolyten, wodurch an dem Filter ein gasförmiges Elektrolyseprodukt entsteht, mit dem der Filter gereinigt wird.

Die Europäische Patentanmeldung mit der Veröffentlichungsnummer 0686 420 Al offenbart ebenfalls ein. Reinigungsverfahren für elektrisch leitfähige Filter, wobei, wie zuvor beschrieben, der Filter als Elektrode einer elektrochemischen Zelle eingesetzt wird. Zum Steigern der Reinigungseffektivität wechselt die Polarität der Elektroden zwischen Reinigungszyklen.

Die Aufgabe der vorliegenden Erfindung besteht darin, eine verbesserte Vorrichtung mit einem elektrolytischen Reinigungsverfahren bereitzustellen.

Die Aufgabe wird erfindungsgemäß gelöst durch die Vorrichtung zur Entnahme von Wasserproben gemäß des unabhängigen Anspruchs 1.

Die erfindungsgemäße Vorrichtung zur Entnahme von Abwasserproben, insbesondere zur fotometrischen Analyse, mit einer Filtervorrichtung, weist eine durch eine Filtermatte (5) abgeschlossene Filterkammer (2) auf, aus der eine Probenentnahmeleitung herausgeführt ist, wobei die Filtermatte (5) mindestens oberflächlich aus elektrisch leitendem Material besteht und als Anode an eine positive elektrische Spannungsquelle angeschlossen ist und wobei im Abstand zur Filtermatte (5) eine an eine negative elektrische Spannungsquelle angeschlossene Kathode (22, 34) angeordnet ist, wobei die Kathode von der Filtermatte elektrisch isoliert ist, wobei ferner die Filterkammer (2) in einem aus elektrisch nicht leitendem Material bestehenden Kammergehäuse (3) ausgebildet ist, das eine von der Filtermatte (5) überspannte Kammeröffnung (4) aufweist, und das Kammergehäuse (3) einen die Kammeröffnung (4) umgebenden, elektrisch leitenden Tragring (24) trägt, gegen den der Rand der Filtermatte (5) durch einen elektrisch nicht leitenden Spannring (25) gedrückt wird, wobei die Kathode entweder als eine die Filtermatte konzentrisch mit Abstand umgebende Ringelektrode oder als eine im Abstand vor der Filtermatte angeordnete Plattenelektrode ausgebildet ist.

Es ist also ein Aspekt der Vorrichtung dass die Filtermatte elektrisch leitend ist bevorzugt und in intermittierend ausgeführten Reinigungsvorgängen zur Durchführung einer anodischen oxidation als Anode mit positivem elektrischem Potential beaufschlagt wird, während eine davon getrennte Kathode mit negativem elektrischen Potential beaufschlagt wird. Die Reinigung der Filtermatte erfolgt durch eine anodische oxidation. Hierbei werden auf elektrochemischem Wege Oxidantien gebildet. Die unmittelbar an der Oberfläche der Filtermatte entstehenden Oxidantien oxidieren den biologischen Bewuchs und verhindern so das Zuwachsen der Filteröffnungen. Das Probenwasser wird dabei ausschließlich einer mechanischen Filtration unterworfen, nicht jedoch einer chemischen oder elektrochemischen Beeinflussung.

Da der Reinigungsvorgang durch Beaufschlagung mit elektrischem Strom erfolgt, kann dieser Reinigungsvorgang besonders vorteilhaft bei Analysegeräten eingesetzt werden, die in Form einer Boje unmittelbar in das zu untersuchende Abwasser eingetaucht werden. Zum Entfernen des biologischen Bewuchses durch anodische Oxidation an der Filtermatte ist es nicht erforderlich, das Analysegerät aus dem Abwasser herauszunehmen und in eine Wartungsposition zu bringen. Deshalb kann die anodische Oxidation in verhältnismäßig geringen Zeitabständen jeweils so rechtzeitig durchgeführt werden, dass eine wesentliche Erhöhung des Durchströmwiderstands der Filtermatte verhindert wird. Damit wird der Energiebedarf gering gehalten. Dies ist insbesondere für Analysegeräte vorteilhaft, die als Boje in das zu untersuchende Abwasser eingetaucht werden (sogenannte In-Situ-Analysatoren).

Gemäß einer vorteilhaften Ausgestaltung des Erfindungsgedankens ist vorgehen, dass die Filtratmenge durch Erzeugen eines Unterdrucks durch die Filtermatte hindurch in eine Filterkammer gesaugt wird und dass der Unterdruck vor und/oder während der Probenentnahme aus der Filterkammer weitestgehend abgebaut wird.

Damit wird erreicht, dass der Energiebedarf für die Entnahme der Abwasserproben aus der Filterkammer und Zuführung zu der Analyseeinrichtung sehr gering ist. Außerdem wird eine Blasenbildung bei der Probenentnahme weitestgehend verhindert, die bei einem verstärkten Unterdruck auftreten würde.

Das zwischen der Filtermatte als Anode und der Kathode befindliche Abwasser dient bei der erfindungsgemäßen Vorrichtung als Elektrolyt. Das Wasser, das der Elektrolyse unterworfen wurde, wird verworfen. Das Abwasser, das der Analyse zugeführt wird, wurde nicht durch einen elektrochemischen Vorgang beeinflusst.

Erfindungsgemäß ist entweder vorgesehen, dass die Kathode eine die Filtermatte konzentrisch mit Abstand umgebende Ringelektrode ist. Damit wird ein besonders einfacher und kompakter Aufbau erreicht.

Oder es kann erfindungsgemäß vorgesehen sein, dass die Kathode eine im Abstand vor der Filtermatte angeordnete Plattenelektrode ist. Dadurch wird ein besonders gleichmäßiges elektrisches Feld zwischen der Filtermatte als Anode und der Kathode ausgebildet, so dass die anodische Oxidation gleichmäßig verteilt in allen Bereichen der Filtermatte erfolgt.

Vorzugsweise besteht die Filtermatte aus Metall. Metall ist elektrisch leitend und hat eine hohe mechanische Festigkeit.

Stattdessen kann die Filtermatte aber auch aus elektrisch nicht leitendem Material, wie Kunststoff oder Keramik, bestehen, das mit einer metallischen Oberflächenbeschichtung versehen ist, die die elektrische Leitfähigkeit an der Oberfläche der Filtermatte gewährleistet.

Weitere vorteilhafte Ausgestaltungen des Erfindungsgedankens sind Gegenstand weiterer Unteransprüche.

Nachfolgend werden Ausführungsbeispiele der Erfindung näher erläutert, die in der Zeichnung dargestellt sind.

Es zeigt:
Fig. 1 eine schematische Darstellung eines Abwasseranalysators mit einer Filtervorrichtung, wobei die erfindungsgemäße Art der Einspannung der Filtermatte, nicht dargestellt ist.
Fig. 2 in einem Längsschnitt durch eine erfindungsgemäße Filtervorrichtung des Analysators nach Fig. 1 und
Fig. 3 in einem Längsschnitt entsprechend der Fig. 2 eine abgewandelte Ausführungsform einer erfindungsgemäßen Filterkammer mit der daran angebrachten Kathode.

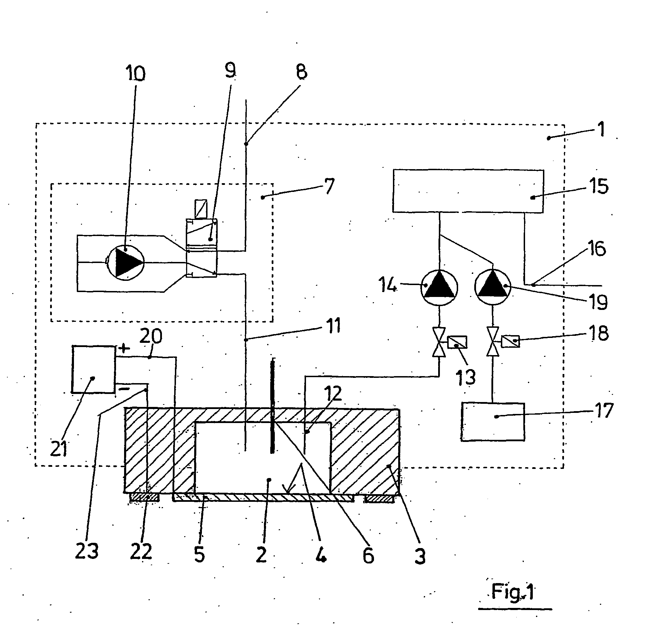

Der in Fig. 1 schematisch dargestellte Abwasseranalysator der Bauart eines In-Situ-Analysators ist als Boje 1 ausgebildet, die in das zu untersuchende Abwasser eingetaucht wird. Eine Filterkammer 2 ist in einem aus elektrisch nicht leitendem Material bestehenden Kammergehäuse 3 ausgebildet. Eine Kammeröffnung 4 der Filterkammer 2 ist mit einer aus Metall bestehenden Filtermatte überspannt, wobei in der Fig. 1 die erfindungsgemäße Art der Einspannung der Filtermatte, nicht dargestellt ist.

In die Filterkammer 2 ragt ein Füllstandssensor 6. Eine Fördereinheit 7 dient dazu, eine Filtratmenge aus dem umgebenden Abwasser durch die Filtermatte 5 hindurch in die Filterkammer 2 anzusaugen. Eine Luftleitung 8 ist über ein Magnetventil 9 und einen elektrisch antreibbaren Kompressor 10 mit einer in der Filterkammer 2 mündenden Saugleitung 11 verbunden.

Eine Probenentnahmeleitung 12 führt aus der Filterkammer über ein Magnetventil 13 und eine Pumpe 14 zu einem fotometrischen Analysator 15, der einen (nicht dargestellten) optischen Sensor aufweist. Von dort führt eine Ablaufleitung 16 aus der Boje 1 hinaus.

Ein Vorratsbehälter 17 für ein Farbreagenz ist über ein Magnetventil 18 und eine Pumpe 19 an die Anschlussleitung des fotometrischen Analysators 15 angeschlossen und dient zur Zugabe eines Farbreagenz zu der zu untersuchenden Abwasserprobe.

Die aus Metall bestehende Filtermatte 5 ist als Anode über eine elektrische Leitung 20 mit dem positivem Ausgang einer elektrischen Gleichspannungsquelle 21 verbunden. Eine die Filtermatte 5 umgebende Ringelektrode 22 ist als Kathode über eine elektrische Leitung 23 mit dem negativem Ausgang der elektrischen Spannungsquelle 21 verbunden.

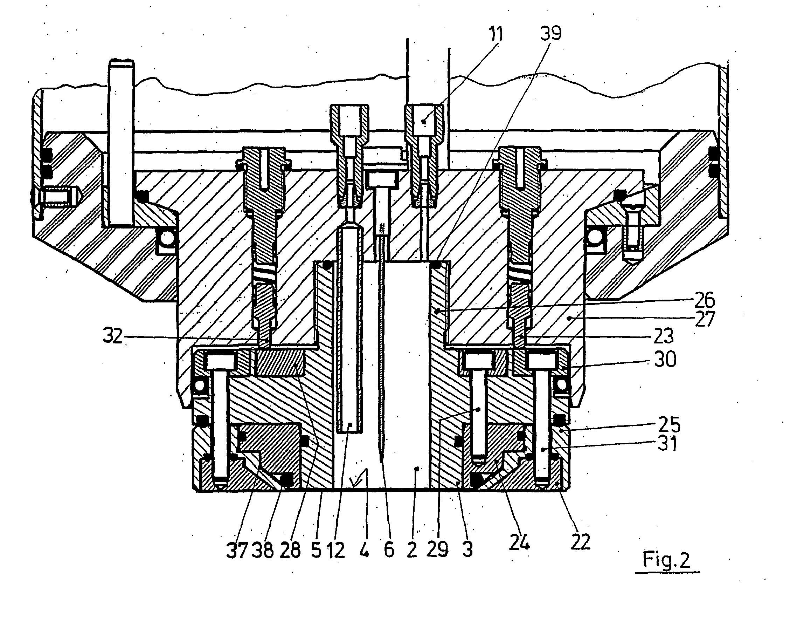

Wie in Fig. 2 in Einzelheiten dargestellt ist, ist die Filterkammer 2 in dem aus elektrisch nicht leitendem Material bestehenden Kammergehäuse 3 ausgebildet, dessen Kammeröffnung 4 von der Filtermatte 5 überspannt wird. Das Kammergehäuse 3 trägt einen die Kammeröffnung 4 umgebenden, elektrisch leitenden Tragring 24, gegen den der Rand der Filtermatte 5 durch einen Spannring 25 aus elektrisch nicht leitendem Material gedrückt wird.

Das Kammergehäuse 3 ist mittels eines am Kammergehäuse 3 einstückig ausgebildeten, zentralen Schraubstutzens 26 auswechselbar an einem Gehäuseboden 27 angeschraubt. Ein geräteseitig am Kammergehäuse 3 angeordneter Schleifring 28 ist mit dem Tragring 24 durch mehrere Spannschrauben 29 mechanisch und elektrisch leitend verbunden. Ein konzentrisch dazu geräteseitig am Kammergehäuse 3 angeordneter Schleifring 30 ist ebenfalls über mehrere achsparallele Spannschrauben 31 mechanisch und elektrisch leitend mit der die Kathode bildende Ringelektrode 22 verbunden. Das Kammergehäuse 3 mit den daran angebrachten Teilen ist somit als Baueinheit leicht auswechselbar am Geräteboden 27 angebracht.

Die beiden Schleifringe 28 und 30 stehen jeweils mit im Geräteboden 27 angeordneten elektrischen Schleifkontakten 32 bzw. 33 in Berührung, die durch den aus elektrisch nicht leitendem Material bestehenden Geräteboden 27 hindurch geführt sind und über die Leitungen 20 bzw. 23 an die elektrische Spannungsquelle 21 angeschlossen sind. Die Verwendung von Schleifringen 28, 30 und Schleifkontakten 32, 33 stellt sicher, dass unabhängig von der jeweils erreichten Einschraubstellung des Kammergehäuses 3 eine elektrische Verbindung zwischen der Filtermatte 5 als Anode und der Ringelektrode 22 als Kathode mit der elektrischen Gleichspannungsquelle 21 gegeben ist.

Die Befüllung der Filterkammer 2 geschieht in der Weise, dass durch die Fördereinheit 7 zunächst ein Unterdruck in der Filterkammer 2 erzeugt wird, so dass eine Filtratmenge des zu untersuchenden Abwassers durch die Filtermatte 5 hindurch in die Filterkammer 2 gesaugt wird. Hierzu wird das in Fig. 1 in seiner stromlosen Stellung gezeigte Magnetventil 9 elektrisch beaufschlagt. Zugleich wird der Kompressor 10 eingeschaltet. Der Kompressor 10 saugt durch die Saugleitung 11 Luft an und drückt sie durch den Luftanschluss 8 nach außen. Dieser Vorgang wird fortgesetzt, bis der Füllstandssensor 6 eine ausreichende Füllung der Filterkammer 2 signalisiert.

Durch Öffnen des Magnetventils 13 und Einschalten der Pumpe 14 wird eine Probenmenge des Filtrats aus der Filterkammer 2 in den fotometrischen Analysator 15 gefördert. Sodann wird durch Öffnen des Magnetventils 18 und Einschalten der Pumpe 19 die jeweils erforderliche Menge an Farbreagenz in den Analysator 15 gefördert. Während der Tätigkeit der Pumpe 14 ist das Magnetventil 9 in seiner in Fig. 1 gezeigten Stellung stromlos. Dadurch ist der Luftweg vom Luftanschluss 8 zur Filterkammer 2 offen. Wenn durch den Saugvorgang der Pumpe 14 ein Unterdruck in der Filterkammer 2 entsteht, so kann Luft über das Magnetventil 9 und den Kompressor 10 nachfließen. Die hierfür erforderliche Druckdifferenz ist auf die verhältnismäßig geringe Kraft begrenzt, die zum Öffnen der Rückschlagventile des Kompressors 10 erforderlich ist. Dadurch erfolgt ein Unterdruckabbau, so dass die Filterkammer 2 angenähert auf dem Druckniveau der Umgebung liegt.

Die Reinigung der Filtermatte 5, insbesondere von biologischem Bewuchs, erfolgt durch eine anodische Oxidation. Durch Beaufschlagung der elektrisch leitenden Filtermatte 5 mit positivem elektrischem Potential durch Einschalten der Gleichstromquelle 21 und Beaufschlagung der Kathode 22 mit negativem elektrischem Potential werden durch Elektrolyse Oxidantien gebildet, wobei die an der Filtermatte als Anode entstehenden Oxidantien den biologischen Bewuchs oxidieren und somit ein Zuwachsen der Filtermatte 5 verhindern. Dieser Reinigungsvorgang durch anodische Oxidation wird intermittierend außerhalb der Probenentnahme durchgeführt, und zwar je nach Bedarf mehrfach zyklisch über den Tag verteilt. Der über die Kathode und Anode geleitete elektrischen Strom und /oder die angelegte Spannung werden dem jeweiligen Reinigungsbedarf angepasst.

Die Reinigungsvorgänge durch anodische Oxidation können zur Zeitoptimierung parallel zum Messbetrieb im Analysator 15 durchgeführt werden.

Zusätzlich zu der beschriebenen Reinigung durch anodische Oxidation kann die Filtermatte 5 je nach Bedarf durch Zuführung eines gesonderten chemischen Reinigungsmittels gereinigt werden.

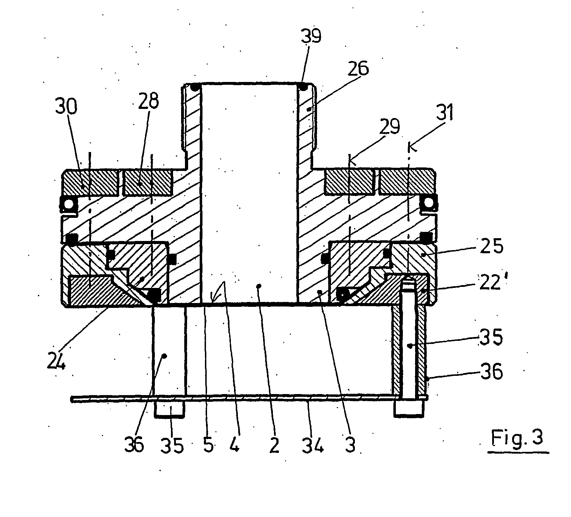

Fig. 3 zeigt eine gegenüber der Fig. 2 abgewandelte Ausführung der Kathode. Während die Kathode bei der Ausführung nach Fig. 2 als eine die Filtermatte 5 konzentrisch mit Abstand umgebende Ringelektrode 22 ausgeführt ist, ist sie bei der Ausführung nach Fig. 3 eine im Abstand vor der Filtermatte 5 angeordnete Plattenelektrode 34, die über mehrere Schrauben 35 und Abstandshülsen 36 mit einem Ring 22' verbunden ist, der der Ringelektrode 22 gleicht.

Neben der beschriebenen Ausführung der Filtermatte 5 aus Metall, beispielsweise Metallgewebe, kann auch vorgesehen sein, die Filtermatte 5 aus elektrisch nicht leitendem Material, beispielsweise Kunststoff oder Keramik, herzustellen und mit einer metallischen Oberflächenbeschichtung zu versehen, um die für die anodische Oxidation erforderliche elektrische Leitfähigkeit zu erreichen.

Wie man aus den Fig. 2 und 3 erkennt, wird der Rand der Filtermatte 5 durch den Spannring 25 gegen eine Kegelstumpffläche 37 des Tragrings 24 gepresst und ist mittels eines Dichtrings 38, beispielsweise eines O-Rings, abgedichtet.

Die Filterkammer 2 ist ein glatt durchgehender, beispielsweise im wesentlichen zylindrischer Hohlraum. Das Kammergehäuse 3 bzw. dessen Schraubstutzen 26 ist im eingeschraubten Zustand mittels einer Dichtung 39 gegenüber dem Gehäuseboden 27 abgedichtet. Dadurch wird eine einfache mechanische Reinigungsmöglichkeit im demontierten Zustand geschaffen.

In die Filterkammer 2 mündet innerhalb des abgedichteten Flächenbereichs des Gehäusebodens 27 die Saugleitung 11. Die Probenentnahmeleitung 12 und der Füllstandssensor 6 ragen aus dem durch die Dichtung 39 umschlossenen Flächenbereich des Gehäusebodens 27 in die Filterkammer 2. Damit wird erreicht, dass beim Auswechseln des Kammergehäuses 3 keine Anschlüsse für diese Leitungen 11, 12 und den Füllstandssensor 6 gelöst werden müssten. Das Auswechseln des die Filtermatte 5 tragenden Kammergehäuses wird dadurch wesentlich erleichtert und vereinfacht, zumal wegen der elektrischen Schleifkontakte 23 und 32 auch keine elektrischen Anschlussleitungen gelöst werden müssen.

## Patentansprüche

1. Vorrichtung zur Entnahme von Abwasserproben, insbesondere zur fotometrischen Analyse, mit einer Filtervorrichtung, die eine durch eine Filtermatte (5) abgeschlossene Filterkammer (2) aufweist, aus der eine Probenentnahmeleitung herausgeführt ist, wobei
die Filtermatte (5) mindestens oberflächlich aus elektrisch leitendem Material besteht und als Anode an eine positive elektrische Spannungsquelle angeschlossen ist, und
im Abstand zur Filtermatte (5) eine an eine negative elektrische Spannungsquelle angeschlossene Kathode (22, 34) angeordnet ist,
wobei die Kathode von der Filtermatte elektrisch isoliert ist, **dadurch gekennzeichnet, dass**
die Filterkammer (2) in einem aus elektrisch nicht leitendem Material bestehenden Kammergehäuse (3) ausgebildet ist, das eine von der Filtermatte (5) überspannte Kammeröffnung (4) aufweist, und
das Kammergehäuse (3) einen die Kammeröffnung (4) umgebenden, elektrisch leitenden Tragring (24) trägt, gegen den der Rand der Filtermatte (5) durch einen elektrisch nicht leitenden Spannring (25) gedrückt wird,
wobei die Kathode
entweder als eine die Filtermatte konzentrisch mit Abstand umgebende Ringelektrode
oder als eine im Abstand vor der Filtermatte angeordnete Plattenelektrode ausgebildet ist.

2. Vorrichtung nach Anspruch 1, **dadurch gekennzeichnet, dass** die Filtermatte (5) aus Metall besteht.

3. Vorrichtung nach Anspruch 1, **dadurch gekennzeichnet, dass** die Filtermatte aus elektrisch nicht leitendem Material, wie Kunststoff oder Keramik besteht, das mit einer metallischen Oberflächenbeschichtung versehen ist.

4. Vorrichtung nach Anspruch 1, **dadurch gekennzeichnet, dass** das Kammergehäuse (3) mit den daran angebrachten Teilen als Baueinheit leicht auswechselbar am Geräteboden (27) angebracht ist.

5. Vorrichtung nach Anspruch 4, **dadurch gekennzeichnet, dass** das Kammergehäuse (3) mittels eines zentralen Schraubstutzens (26) auswechselbar an einem Gehäuseboden (27) angeschraubt ist, dass ein mit dem Tragring (24) elektrisch leitend verbundener Schleifring (28) und ein mit der Kathode (22 bzw. 34) elektrisch leitend verbundener Schleifring (30) geräteseitig am Kammergehäuse (3) konzentrisch angeordnet sind und jeweils mit im Geräteboden (27) angeordneten elektrischen Schleifkontakten (32 bzw. 33) in Berührung stehen.

6. Vorrichtung nach Anspruch 4 oder 5, **dadurch gekennzeichnet, dass** die Filterkammer (2) ein glatt durchgehender, beispielsweise im wesentlichen zylindrischer Hohlraum ist, und dass das Kammergehäuse (3) bzw. dessen Schraubstutzen (26) im eingeschraubten Zustand mittels einer Dichtung (39) gegenüber dem Gehäuseboden (27) abgedichtet ist.

## Claims

1. Device for taking wastewater samples, particularly for photometric analysis, with a filter unit that exhibits a filter chamber (2) sealed by a filter mesh (5) from which leads a line for removing the samples **characterized in that**
the filter mesh (5) consists of electrically conductive material, at least on its surface, and is connected to a positive electrical voltage source as an anode and
a cathode (22, 34), connected to a negative electrical source, is arranged at a distance away from the filter mesh (5) whereby said cathode is electrically isolated from the filter mesh **characterized in that**
the filter chamber (2) is formed by a chamber housing (3) consisting of electrically non-conductive material and the chamber housing (3) exhibits a chamber opening (4) covered by a tensioned filter mesh (5)
and **in that** the chamber housing (3) bears an electrically conductive support ring (24) surrounding the chamber opening (4) against which the edge of the filter mesh (5) is pressed by a clamping ring (2) made of electrically non-conductive material
whereby the cathode is either designed a) as an annular electrode concentrically encompassing the filter mesh at a distance or b) as a plate electrode arranged at a distance from the filter mesh.

2. Device as per Claim 1 **characterized in that** the filter mesh (5) is made of metal.

3. Device as per Claim 1 **characterized in that** the filter mesh comprises electrically non-conductive material, such as plastic or ceramic, that is provided with a metallic surface coating.

4. Device as per Claim 1 **characterized in that** the chamber housing (3) together with its attached parts is arranged on the device bottom (27) as a component that can be easily interchanged.

5. Device as per Claim 4 **characterized in that** the chamber housing (3) is screwed onto the device floor (27) by means of a central screw socket (26), such that the chamber housing can be interchanged, and **in that** a first electrically conductive slip ring (28) connected to the support ring (24) and a second electrically conductive slip ring (30) connected to the cathode (22 or 34) are concentrically arranged on the chamber housing (3) on the device side and are in contact with electrical sliding contacts (32 and 33) arranged in the device bottom (27).

6. Device as per Claim 4 or 5 **characterized in that** the filter chamber (2) is a smooth, continuous, essentially cylindrical cavity and that the chamber housing (3) or its screw socket (26) is sealed in its screw-in condition against the housing bottom (27) by means of a seal (39).

## Revendications

1. Dispositif destiné au prélèvement d'échantillons d'eaux usées, notamment pour analyse photométrique, équipé d'un dispositif de filtrage, qui comporte une chambre de filtrage (2) fermée par une toile filtrante (5), chambre depuis laquelle une conduite de prélèvement d'échantillons est sortie,
la toile filtrante (5) étant constituée au moins en surface d'une matière électroconductrice et étant raccordée en tant qu'anode à une source de tension électrique positive, et
une cathode (22, 34) raccordée à une source de tension électrique négative étant disposée à distance de la toile filtrante (5),
la cathode étant isolée électriquement de la toile filtrante, **caractérisé en ce que**
la chambre de filtrage (2) est formée dans un corps de chambre (3) constitué d'une matière non électroconductrice, corps qui présente une ouverture de chambre (4) recouverte par la toile filtrante (5), et **en ce que**
le corps de chambre (3) supporte une bague-support (24) électroconductrice enveloppant l'ouverture de chambre (4), bague-support contre laquelle le bord de la toile filtrante (5) est pressé par une bague de serrage (25) non électroconductrice,
la cathode étant
soit constituée d'une électrode annulaire enveloppant à distance la toile filtrante de façon concentrique
soit constituée d'une électrode à plaque disposée à distance devant la toile filtrante.

2. Dispositif selon la revendication 1, **caractérisé en ce que** la toile filtrante (5) est en métal.

3. Dispositif selon la revendication 1, **caractérisé en ce que** la toile filtrante est constituée d'une matière non électroconductrice, par exemple en plastique ou en céramique, qui est pourvue d'un revêtement de surface métallique.

4. Dispositif selon la revendication 1, **caractérisé en ce que** le corps de chambre (3) avec les pièces accolées est monté sur la partie inférieure de l'appareil (27) en tant qu'unité modulaire facilement interchangeable.

5. Dispositif selon la revendication 4, **caractérisé en ce que** le corps de chambre (3) est vissé de façon interchangeable sur une partie inférieure de corps (27) au moyen d'un raccord fileté central (26), **en ce qu'**une bague glissante (28) reliée de façon électroconductrice avec la bague-support (24) et une bague glissante (30) reliée de façon électroconductrice avec la cathode (22 ou 34) sont disposées de façon concentrique côté appareil sur le corps de chambre (3) et sont chacune en contact avec des contacts électriques glissants (32 ou 33) disposés dans la partie inférieure d'appareil (27).

6. Dispositif selon la revendication 4 ou 5, **caractérisé en ce que** la chambre de filtrage (2) est une cavité entièrement traversante, par exemple essentiellement cylindrique, et **en ce que** le corps de chambre (3) ou son raccord fileté (26) est rendu étanche à l'état vissé au moyen d'un joint d'étanchéité (39) par rapport à la partie inférieure de corps (27).
